# EUROPÄISCHE PATENTSCHRIFT

(11) **EP 2 110 664 B1**
(45) Veröffentlichungstag und Bekanntmachung des Hinweises auf die Patenterteilung: **24.08.2011**
(21) Anmeldenummer: 08154804.2
(22) Anmeldetag: 18.04.2008
(51) Int. Cl.: G01N 33/18, B01D 3/34

(54) **Temperierung eines Messgutes in einem Stripper durch direkte Dampfinjektion**
Tempering of a medium to be measured in a stripper by means of direct steam injection
Dispositif destiné à tempérer un produit de mesure dans un dispositif de stripage par injection de vapeur directe

(43) Veröffentlichungstag der Anmeldung: 21.10.2009
(73) Patentinhaber: BASF SE, 67056 Ludwigshafen (DE)
(72) Erfinder: Schocker, Alexander, 68163 Mannheim (DE); Krohn, Sven, 67105 Schifferstadt (DE); Lißner, Bert, 67063 Ludwigshafen (DE); Kuhl, Siegfried, 67434 Neustadt (DE)

(56) Entgegenhaltungen:
- DE-U1- 8 804 409
- US-A- 3 386 891
- US-A- 3 441 483
- US-A- 4 154 086
- US-A- 4 737 467
- US-B1- 6 436 710
- US-B1- 6 485 688
- H. BORÉN ET AL.: "Optimization of the Open Stripping System for the Analysis of Trace Organics in Water" JOURNAL OF CHROMATOGRAPHY, Bd. 348, 1985, Seiten 67-78, XP002497795 Amsterdam
- "Absorption and Stripping of Dilute Mixtures" In: J.D.Seader, E.J. Henley: "Separation Process Principles", 1998, John Wiley & Sons ISBN: 0-471-58626-9 pages 270-271,
- B. Lissner: "Thermostatisieren von Abwassermessungen mit Hilfe eines direkt dampfbeheizten Strippsystems", 2006, Berufsbildende Schule Naturwissenschaften Ludwigshafen page 17,33,35,

## Beschreibung

Die vorliegende Erfindung betrifft ein Verfahren zur Analyse eines Messgutes, wobei das Messgut einem Stripper zugeführt wird, mindestens ein Strippmedium in das Messgut eingebracht wird, Komponenten zumindest teilweise aus dem Messgut in das Strippmedium übergehen und das so erhaltene Messgas in eine Analyseeinrichtung geleitet wird. Weiterhin betrifft die Erfindung eine Vorrichtung zum Strippen von Komponenten aus einem Messgut umfassend ein Grundelement mit mindestens einem Einlass und mindestens einem Auslass für das Messgut, mindestens einem Einlass für mindestens ein Strippmedium, mindestens einem Auslass für Messgas, das sich aus Strippmedium und aus dem Messgut ausgestrippten Komponenten zusammensetzt, eine Analyseeinrichtung, in die das Messgas geleitet wird, mindestens einem weiteren Einlass für ein Temperiermedium oder mehrere Temperiermedien und mindestens einem Messfühler zur Aufnahme der Temperatur des Messgutes. Bevorzugte Ausführungsformen sind den Unteransprüchen und der Beschreibung zu entnehmen. Vom Rahmen der vorliegenden Erfindung werden selbstverständlich auch Kombinationen bevorzugter Ausführungsformen umfasst.

Unter "Strippen" wird in der Verfahrenstechnik üblicherweise ein Prozess verstanden, bei dem ein Gas durch eine Flüssigkeit geleitet wird, wobei einige Komponenten aus der Flüssigkeit in die Gasphase übergehen. Ob und in welchem Maße Komponenten in die Gasphase übergehen, hängt im Wesentlichen von den physikalischen und chemi-schen Eigenschaften der Substanzen in Flüssig- und Gasphase sowie den Prozessbedingungen wie Druck und Temperatur ab.

Als Strippvorrichtungen oder "Stripper" werden häufig Kolonnen oder Säulen eingesetzt, die meist in vertikaler Richtung röhrenförmig ausgebildet sind. In der Betriebsweise wird zwischen Gleich- und Gegenstromprinzip einerseits sowie kontinuierlichem und diskontinuierlichem Betrieb andererseits unterschieden. Beim Gleichstromprinzip wird das gasförmige Strippmedium in Fließrichtung mit dem Flüssigkeitsstrom geleitet, beim Gegenstromprinzip bewegen sich die Ströme in entgegengesetzter Richtung. Beim kontinuierlichen Betrieb strömen Flüssigkeit und Gas kontinuierlich durch die Strippvorrichtung. Beim diskontinuierlichen Betrieb hingegen wird beispielsweise eine Menge an Flüssigkeit in der Strippvorrichtung vorgelegt, durch die anschließend über eine gewisse Zeitspanne ein gasförmiges Strippmedium strömt.

Strippvorrichtungen werden für Stofftrennungen zu unterschiedlichen Zwecken eingesetzt. Beispielsweise werden industrielle Abwässer in großtechnischen Anlagen kontinuierlich aufbereitet und gereinigt. Stripper werden auch eingesetzt, um in prozesstechnischen Anlagen Wertprodukte zu gewinnen oder zu reinigen. Häufig handelt es sich dabei um Zwischenschritte bei mehrstufigen Verfahren. Ein Beispiel aus der Petrochemie ist das Dampfstrippen von Naphtha- und Kerosinfraktionen bei der Rohöldestillation. Ein weiteres Einsatzgebiet von Strippvorrichtungen ist die Aufbereitung von Proben zu Analysezwecken. In Kombination mit einer oder mehreren Analyseeinrichtungen werden Stripper beispielsweise zur Überwachung von Abwasser, Kühlwasser oder auch Produktströmen in verfahrenstechnischen Anlagen eingesetzt. Ein Ziel ist dabei, Verunreinigungen der genannten Medien, z.B. durch Kohlenwasserstoffe im Spurenbereich (ppm) nachzuweisen.

Die Palette an nachweisbaren Substanzen erstreckt sich über einen weiten Bereich von organischen Verbindungen wie leicht- bis schwersiedende Kohlenwasserstoffe, unpolare und polare Kohlenwasserstoffe, z.B. Aromaten, Ester, Alkohole, Amine, Phenole, bis hin zu anorganischen Verbindungen wie Schwefelwasserstoff oder Ammoniak. Die Substanzen können unterschiedliche Löslichkeiten in Wasser aufweisen. Der Strippvorgang ist stark temperaturabhängig. Das Austreiben einzelner Substanzen geschieht bei verschiedenen Temperaturen unterschiedlich effizient. Einige Substanzen lassen sich erst bei höheren Temperaturen signifikant ausstrippen. Der Artikel "Optimization of the open stripping system for the analysis of trace organics in water" von Borén et al. (Journal of Chromatography, 348 (1985), S. 67-78) zeigt die Ergebnisse des Ausstrippens von verschiedenen polaren und unpolaren Substanzen unterschiedlicher Volatilität aus Wasser bei 30°C, 60°C und 90°C. Durch Temperaturschwankungen im Messgut, in obigem Beispiel verunreinigtes Wasser, oder in der Umgebung des Strippers und den Analyseeinrichtungen können erhebliche Ungenauigkeiten bei der Bestimmung der einzelnen Substanzen auftreten. Auch natürliche Änderungen der Umgebungstemperatur im jahreszeitlichen Verlauf und im Tag-Nacht-Zyklus können die Analyseergebnisse erheblich beeinflussen.

Insbesondere für Strippvorrichtungen, die zu Analysezwecken eingesetzt werden, sind Möglichkeiten bekannt, das Messgut zu temperieren, um die Auswirkungen von Temperaturschwankungen abzuschwächen oder gänzlich auszugleichen.

In US 4,154,086 wird eine Vorrichtung und ein Verfahren offenbart zur Detektion von Spuren flüchtiger organischer Substanzen in Wasser. Die Vorrichtung besteht aus einer Kolonne, in die im unteren Teil Wasser im Gegenstrom zu einem Strippgas geführt wird. Das Wasser wird über einen Wärmetauscher temperiert, der beispielhaft als von Dampf durchströmte Heizschlange ausgeführt ist. Das aufsteigende Gas, das aus dem Strippgas, ausgestrippten organischen Komponenten und eventuell Anteilen von Wasser besteht, wird durch einen Wärmetauscher geleitet, der so betrieben wird, dass die Wasseranteile auskondensieren. Der verbleibende Gasstrom wird einer Analyseeinrichtung zugeführt.

US 6,485,688 beschreibt einen Gegenstrom-Stripper zur Abtrennung von polaren und unpolaren flüchtigen organischen Substanzen aus Abwasser. Der Stripper besteht aus einer Säule, an deren unterem Ende sich ein u-formiger Abschnitt anschließt, der so gestaltet ist, dass sich aufgrund des hydrostatischen Drucks im Stripper eine Flüssigkeitssäule ausbildet. In den unteren Bereich des Strippers wird ein Strippgas eingebracht, das entgegen der Fließrichtung der Flüssigkeit nach oben strömt. Dabei werden flüchtige Komponenten aus der Abwasserprobe ausgestrippt und mit dem Strippgas aus einer Öffnung im oberen Teil des Strippers zu Analyseeinrichtungen geleitet. Die Möglichkeit der Erwärmung des Messgutes wird allgemein erwähnt, es finden sich allerdings keine konkreten Hinweise auf die Ausgestaltung einer Heizung.

US 6,436,710 beschreibt ebenfalls eine Vorrichtung und ein Verfahren zur Analyse von flüchtigen organischen Substanzen in einer Abwasserprobe. Dabei wird ein Inertgas in eine flüssige Probe geleitet, löst dabei flüchtige Komponenten aus der Flüssigkeit und überführt sie in die Gasphase. Im Gasraum ist ein Sensor angebracht, der Messinformationen über die flüchtigen organischen Komponenten an eine Analyseeinrichtung weiterleitet. Die flüssige Probe kann durch übliche Heizvorrichtungen erhitzt werden.

In US 4,737,467 werden eine Vorrichtung und ein Verfahren beschrieben zum Austreiben von flüchtigen organischen Substanzen aus einer wässrigen Phase zur Vorbereitung einer Analyse mittels eines Gaschromatographen. Zur Verbesserung des Stoffübergangs der flüchtigen Substanzen von der Flüssig- in die Gasphase umfasst die Vorrichtung einen elektromechanischen Antrieb, mit dem der Behälter, in dem sich die wässrige Phase befindet, in Schwingungen versetzt wird. Um die wässrige Phase erwärmen zu können, ist ein Gebläse vorgesehen, das heiße Luft von außen auf die Behälterwand leitet.

In dem deutschen Gebrauchsmuster DE 8804409 wird ein Gerät offenbart, das geeignet ist, flüchtige organische Stoffe aus einer flüssigen Phase, beispielsweise Abwasser, zu extrahieren und einer Analyse zugänglich zu machen. Das Gerät kann mit einer Temperaturregelung versehen sein, die so betrieben werden kann, dass das Messgut während des Strippprozesses auf einer konstanten Temperatur gehalten wird. Als Mittel zum Heizen ist beispielhaft eine elektrische Heizung offenbart.

In DE 601 09 191 wird eine Vorrichtung und ein Verfahren zur kontinuierlichen Behandlung von Industrieabwasser durch Strippung mit Wasserdampf beschrieben. Bei der Vorrichtung handelt es sich um eine Strippsäule, bei der das Abwasser im oberen Teil und der Wasserdampf im unteren Teil der Säule zugeführt werden. Die Zuläufe und das Rücklaufverhältnis am Kopf der Säule können über Stellorgane so beeinflusst werden, dass eine im Zu- und/ oder Ablauf des Abwassers gemessene Konzentration an interessierenden Komponenten auf einen gewünschten Wert eingestellt wird.

US 3,441,483 offenbart ein Verfahren zur Regelung der Temperatur in einer Trennoder Strippkolonne. Ziel ist dabei die selektive Abtrennung von leicht flüchtigen Komponenten aus einem Gemisch, das der Kolonne zugeführt wird. Um die Effizienz der Abtrennung zu erhöhen, wird ein flüssiges Absorbens oberhalb des Feedzulaufs auf die Kolonne gegeben, das im Gegenstrom zu dem die abzutrennenden Komponenten enthaltenden Dampf nach unten strömt. Der Sumpfaustrag aus der Kolonne wird verdampft und ein Teil dieses Dampfes der Kolonne wieder zugeführt. Dieser Dampfstrom wird verwendet, um eine möglichst konstante Temperatur auf einem ausgewählten Boden der Kolonne zu gewährleisten.

Der im Folgenden beschriebenen Erfindung lag die Aufgabe zugrunde, eine Vorrichtung und ein Verfahren zu entwickeln, mit Hilfe derer ein Messgut in einem Stripper schnell und effizient auf eine gewünschte Temperatur temperiert werden kann. Die Vorrichtung sollte konstruktiv einfach und kostengünstig im Betrieb sein. Dabei sollten das Verfahren und die Vorrichtung sowohl für kontinuierlichen als auch für diskontinuierlichen Betrieb geeignet sein. Das Verfahren und die Vorrichtung werden auf dem Gebiet der Analytik eingesetzt.

Zur Lösung dieser Aufgabe wird ein Verfahren vorgeschlagen zur Analyse eines Messgutes, wobei das Messgut einem Stripper zugeführt wird, mindestens ein Strippmedium in das Messgut eingebracht wird, Komponenten zumindest teilweise aus dem Messgut in das Strippmedium übergehen und das so erhaltene Messgas in eine Analyseeinrichtung geleitet wird, wobei das Messgut temperiert wird, indem Dampf mindestens eines Temperiermediums direkt in das Messgut eingebracht wird.

Weiterhin wird eine Vorrichtung zum Strippen wie eingangs beschrieben vorgeschlagen.

Unter Temperierung wird im Folgenden der Vorgang verstanden, eine Substanz oder eine Mischung von Substanzen auf eine gewünschte Temperatur zu bringen. Erfindungsgemäß erfolgt die Temperierung eines Messgutes dadurch, dass direkt in das Messgut ein Temperiermedium eingebracht wird oder mehrere Temperiermedien eingebracht werden. Das Temperiermedium wird oder Temperiermedien werden dampfförmig zugeführt. Die Begriffe "dampfförmig" und "gasförmig" bzw. "Dampf" und "Gas" werden im Folgenden als gleichwertig angesehen.

Besonders bevorzugt wird das Temperiermedium oder werden die Temperiermedien direkt in ein im Wesentlichen flüssiges Messgut eingeleitet. Bei mehreren Temperiermedien kann es sich zum einen um eine einzige Substanz handeln, die über mehrere Zuläufe zugeführt wird. Zum anderen werden damit auch unterschiedliche Substanzen bezeichnet, die über einen oder mehrere Zuläufe eingebracht werden. Wird das Temperiermedium oder werden die Temperiermedien dampfförmig in das Messgut eingebracht, so kondensiert der Dampf in Abhängigkeit von Druck- und Temperaturbedingungen teilweise oder vollständig und gibt Wärme an das Messgut ab. Das entstehende Kondensat vermischt sich mit der flüssigen Phase des Messgutes. Selbstverständlich kann das Messgut zusätzlich zur Temperierung mittels Einbringen von einem oder mehreren Temperiermedien auf konventionelle Art temperiert werden, beispielsweise durch eine Beheizung über die Wandung des Strippers, durch Heizelemente im Innern des Strippers oder dem Stripper vorgeschaltete Wärmetauscher. Oft ist es jedoch hinreichend, die Temperierung nur mittels Einbringen eines oder mehrerer Temperiermedien in das Messgut vorzunehmen.

Das Messgut kann aus unterschiedlichen Substanzen bestehen. Beispielsweise kann das Messgut zum Großteil aus einer Hauptkomponente bestehen, die mit verschiedenen weiteren Substanzen vermischt ist, und von denen zumindest einige teilweise oder vollständig durch Strippen aus der Hauptkomponente entfernt werden können. Bevorzugt handelt es sich bei dem Messgut im Wesentlichen um Wasser, das mit organischen oder anorganischen Verbindungen verunreinigt sein kann. Das Messgut kann auch ein Produkt eines verfahrenstechnischen Prozesses sein, das noch Nebenprodukte oder sonstige Substanzen enthält. Das Messgut ist im Wesentlichen flüssig, wenn es mit dem Temperiermedium in Kontakt gebracht wird. "Im Wesentlichen" bedeutet in diesem Zusammenhang, dass auch andere Phasen in der Flüssigphase enthalten sein können, beispielsweise gelöste oder ungelöste Gaspartikel oder auch Feststoffe wie Schwebeteilchen oder Kristalle.

Bei dem Strippmedium handelt es sich bevorzugt um ein Gas oder Gasgemisch, das geeignet ist, nachzuweisende Substanzen aus dem Messgut aufzunehmen. Das Strippmedium sollte keine Substanzen enthalten, die die Analyse der nachzuweisenden Substanzen verfälschen können. Bevorzugt sind beispielsweise Luft oder Stickstoff, die sich gegenüber den nachzuweisenden Substanzen inert verhalten. In Abhängigkeit von den zur Analyse eingesetzten Einrichtungen und Methoden erweist sich insbesondere gereinigte Luft als vorteilhaftes Strippmedium. Die Reinigung der Luft kann beispielsweise katalytisch erfolgen. In Abhängigkeit von dem Messgut und den nachzuweisenden Substanzen kann als Strippmedium auch ein Gas oder Gasgemisch eingesetzt werden, das chemisch mit einer oder mehreren Substanzen reagiert und diese in eine oder mehrere andere Substanzen umwandelt. Diese Ausführungsform eines Strippmediums ist insbesondere für solche Substanzen vorteilhaft, die sich selbst nur schwer in einer Analyseeinrichtung erfassen lassen, deren Umsetzungsprodukte, die durch die Reaktion mit dem Strippmedium entstehen, dagegen leichter erfassbar sind. Außerdem lässt sich diese Ausführungsform vorteilhaft anwenden, wenn eine Substanz analysiert werden soll, die sich nicht oder nur schwer aus dem Messgut ausstrippen lässt, deren Reaktionsprodukt aber leichter in die Gasphase übergeht.

Durch den Stoffaustausch zwischen Messgut und Strippmedium gehen Komponenten zumindest teilweise aus dem Messgut in die Gasphase über und vermischen sich mit dem Strippmedium. Das so entstehende Messgas kann einer Analyseeinrichtung zugeleitet werden. Prinzipiell lassen sich verschiedenste Analyseeinrichtungen und Analysemethoden zum Nachweis verwenden. Welche Analyseeinrichtung eingesetzt wird bzw. welche Analysemethoden verwendet werden, hängt von den zu bestimmenden Parametern ab. Verbreitet ist die Detektion von Substanzen z.B. mittels Summen-Flammenionisationsdetektoren (FID), Gaschromatographie, Photometrie, Spektroskopie oder Gasdetektoren. Es können auch mehrere Analyseeinrichtungen oder Kombinationen von Analyseeinrichtungen Verwendung finden. Das erfindungsgemäße Verfahren und die erfindungsgemäße Vorrichtung lassen sich vorteilhaft anwenden, um eine breite Palette an Substanzen aus einem Messgut auszustrippen, beispielsweise die eingangs erwähnten, z.B. Cyclohexan, Tetrahydrofuran oder Aceton, Alkohole wie Methanol, Ethanol, Butanol oder Propanol, Acrylate wie Methylacrylat oder Ethylacrylat, Aromaten wie Ethylbenzol, aber auch anorganische Verbindungen wie Schwefelwasserstoff oder Ammoniak. Die Substanzen können dabei in Konzentrationen von mehreren Prozent bis in den Spurenbereich, also wenige ppm oder ppb, einzeln oder in Summe im Messgut vorhanden sein.

Als Temperiermedien kommen unterschiedliche Stoffe oder Stoffgemische in Betracht. Sie sollten so gewählt werden, dass sie sich mit dem Messgut schnell vermischen und keine Substanzen enthalten, die die Analyse des Messgases verfälschen können. Besteht das Messgut beispielsweise im Wesentlichen aus Wasser mit Verunreinigungen durch organische und/oder anorganische Verbindungen, so eignet sich Wasserdampf als Temperiermedium gut. Bei dem Wasserdampf handelt es sich vorteilhaft um Prozessdampf, wie er in Anlagen der chemischen Industrie üblicherweise in verschiedenen Druckstufungen zur Verfügung steht. Geeignete Druckstufungen sind z.B. (Druck als Absolutangaben):
1 bis 2 bar, 120 bis 150 °C
4 bis 5 bar, 130 bis 200 °C
15 bis 18 bar, 200 bis 300 °C
40 bis 45 bar, 250 bis 300 °C
50 bis 55 bar, 260 bis 300 °C

Selbstverständlich können erfindungsgemäß auch Temperiermedien bei anderen Drücken und Temperaturen eingesetzt werden, solange sie in der Lage sind, Wärme an das Messgut abzugeben.

Wenn es sich bei dem Messgut nicht im Wesentlichen um Wasser, sondern beispielsweise einen Produktstrom aus einer verfahrenstechnischen Anlage handelt, so eignen sich besonders Dämpfe des nahezu reinen Produktes als Temperiermedium. Es können auch andere Stoffe oder Stoffgemische eingesetzt werden, die sich mit dem Produkt gut und schnell mischen und keine Substanzen einbringen, die die Analyseergebnisse verfälschen können.

Die erfindungsgemäße Strippvorrichtung, im Folgenden auch kurz als Stripper bezeichnet, kann unterschiedlich ausgestaltet sein. Allgemein enthält die Vorrichtung ein Grundelement, das ein oder mehrere, vorzugsweise röhrenförmige Bauteile mit mindestens einem Einlass und mindestens einem Auslass für ein Messgut umfasst. Der Querschnitt der Bauteile kann unterschiedlich ausgeführt sein, beispielsweise quadratisch, rechteckig oder oval. Bevorzugt ist der Querschnitt im Wesentlichen kreisförmig bis völlig rund. Die Querschnittsformen sowie die Durchmesser können für die jeweiligen Bauteile unterschiedlich sein. Häufig ist der Stripper im Wesentlichen vertikal angeordnet. Der Stripper umfasst des Weiteren mindestens einen Messfühler zur Aufnahme der Temperatur des Messgutes.

Weiterhin weist der Stripper mindestens einen Einlass für ein Strippmedium sowie mindestens einen Auslass für ein Messgas auf. Um das Messgut auf eine gewünschte Temperatur bringen zu können, ist mindestens ein weiterer Einlass für mindestens ein Temperiermedium vorgesehen. Sämtliche Zu- und Abläufe, beispielsweise für Messgut, Strippmedium, Temperiermedium oder Messgas, können mit Einrichtungen versehen sein, mit deren Hilfe die jeweiligen Durchflüsse beeinflusst werden können. Beispiele für derartige Stellorgane sind Kugelhähne, Stellklappen oder Stellventile. Erfindungsgemäß bevorzugt ist in mindestens einem Zulauf eines Temperiermediums ein Stellorgan angebracht, wodurch sich der Zufluss an Temperiermedium in einem Bereich von null bis zu einem Maximalwert variieren lässt. Der Maximalwert ist abhängig von der konkreten Ausgestaltung und Geometrie des Stellorgans sowie der Druckdifferenz über das Stellorgan.

Der Eintritt des Temperiermediums oder der Temperiermedien in den Stripper kann unterschiedlich realisiert sein. In einer einfachen Ausführungsform eines Einlassstutzens taucht ein Rohr mit dem offenen Ende in die Flüssigphase des Strippers. Eine weitere Ausführungsform stellt ein Rohr dar, das in die Flüssigphase des Strippers eintaucht, wobei der in die Flüssigkeit eintauchende Teil des Rohres mehrere Öffnungen aufweist, aus denen das Temperiermedium ausströmt. Bevorzugt sind Einlassstutzen, die eine gleichmäßige und feine Verteilung des Dampfes sicherstellen und damit eine schnelle und effiziente Erwärmung des Messgutes ermöglichen, beispielsweise Düsen. Besonders bevorzugte Ausführungsformen stellen Dampfinjektoren dar. Sie bestehen im Wesentlichen aus einem Gehäuse, in dem sich eine Venturi-Düse sowie ein konisch ausgebildeter Diffusor befinden. Die beiden Bauteile sind so angeordnet, dass sie einen Ringspalt bilden, dessen Fläche abhängig von der anliegenden Druckdifferenz den Dampfdurchsatz durch den Injektor begrenzt. Der Dampf tritt tangential aus dem Ringspalt in das Messgut aus, wobei er kondensiert. Der Auslass ist derart gestaltet, dass Verwirbelungen entstehen, die für eine schnelle Durchmischung des Temperiermediums mit dem Messgut sorgen. Die Verwirbelungen fördern zudem das Ausstrippen der Komponenten von der flüssigen in die gasförmige Phase.

Der erfindungsgemäße Stripper kann weitere Einlässe aufweisen, beispielsweise zur Zufuhr einer Substanz, mit Hilfe derer in dem oder den verwendeten Analyseeinrichtungen eine Kalibrierung durchgeführt werden kann. Auch eine Referenz- oder Markierungssubstanz kann durch einen oder mehrere solcher zusätzlichen Einlässe in das Messgut oder das Messgas eingebracht werden. Eine solche Substanz kann auch einem Strippmedium oder einem Temperiermedium zugeführt werden und mit diesen Medien in den Stripper gelangen.

Liegt ein Medium in einem Zulauf nicht bei einem höheren Druck vor als er im Stripper vorherrscht, wird in aller Regel eine Fördereinrichtung eingesetzt, um diesen Druckunterschied zu überwinden und das Medium in den Stripper zu fördern. In einem solchen Fall kann ein Stellorgan auch in die jeweilige Fördereinrichtung integriert sein, beispielsweise in Form einer Dosierpumpe.

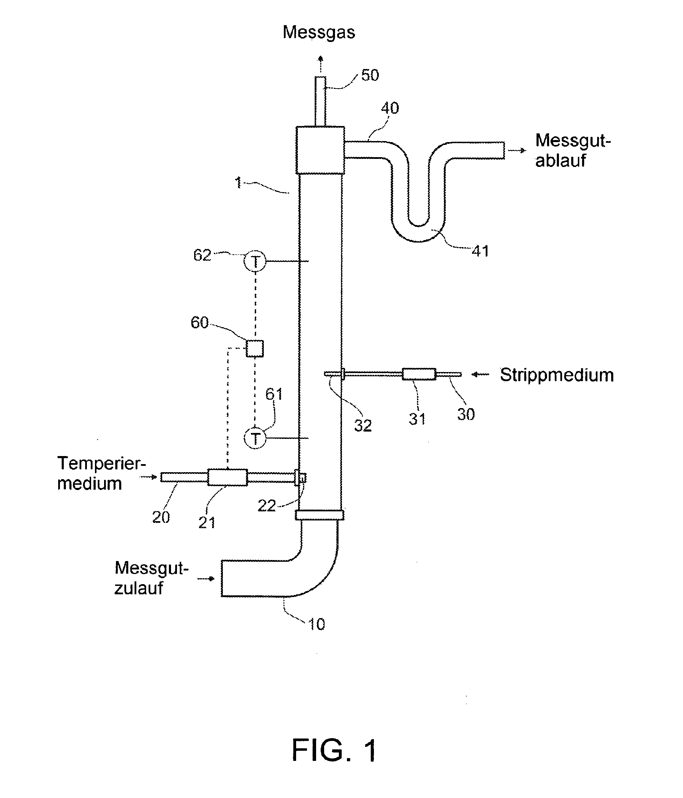

Ein- und Auslässe für Zu- und Abläufe können an unterschiedlichen Stellen am Stripper angebracht sein. Einige vorteilhafte Kombinationen sind unten stehend in den Beschreibungen der Fig. 1 bis 4 näher ausgeführt. In einer besonders bevorzugten Ausführungsform umfasst das Grundelement des Strippers ein oder mehrere rohrförmige Bauteile. Das Grundelement ist vorteilhaft im Wesentlichen vertikal angeordnet. Am unteren Ende des Grundelementes ist ein Einlass für ein Messgut vorgesehen. Oberhalb des Messguteinlasses ist mindestens ein Einlass für ein Temperiermedium vorhanden. Oberhalb des Temperiermedium-Einlasses wird ein Strippmedium durch mindestens einen Einlass eingebracht. Das Strippmedium kann auch zwischen Messguteinlass und Temperiermedium-Einlass zugeführt werden. Am oberen Ende des Grundelementes verlässt das Messgut das Grundelement über einen Auslass, vorteilhaft durch eine Leitung, die als Siphon ausgeführt ist. Noch oberhalb des Messgutauslasses ist am Grundelement ein Auslass für das Messgas vorhanden.

Die Temperatur des Messgutes wird bevorzugt mit Hilfe eines oder mehrerer Temperatur-Messfühler erfasst. Auf der Basis der Information über die Temperatur im Messgut wird das Stellorgan oder werden die Stellorgane eines Zulaufs oder mehrerer Zuläufe an Temperiermedium so eingestellt, dass sich die Temperatur im Messgut einem gewünschten Sollwert annähert. In einer bevorzugten Ausführungsform werden Informationen aus dem Temperatur-Messfühler oder den Temperatur-Messfühlern einer Regeleinrichtung zugeführt. Die Regeleinrichtung kann unterschiedlich ausgeführt sein, beispielsweise analog oder digital, als eigenständiges Bauteil oder kombiniert mit einem Messfühler, einem Stellorgan oder beidem. Regelprinzipien und -algorithmen sind dem Fachmann bekannt. In einer besonders bevorzugten Ausführungsform einer Regeleinrichtung wird aus Messinformationen und einem gegebenen Sollwert für die Temperatur ein Signal für das Stellorgan im Zulauf oder Signale für Stellorgane in mehreren Zuläufen des Temperiermediums bestimmt. Die Messinformationen können aus unterschiedlichen Arten von Messfühlern stammen. Bevorzugt wird mittels mindertens eines Temperatur-Messfühlers die Temperatur im Messgut erfasst. Dies kann bereits im Zulauf des Messgutes erfolgen oder an unterschiedlichen Stellen im Grundelement des Strippers. Neben den Messfühlern zur Temperaturerfassung können weitere Messfühler vorhanden sein, beispielsweise zur Aufnahme des Drucks oder Durchflusses an unterschiedlichen Medien. Die Signale dieser Messfühler können einer oder mehreren Regeleinrichtungen ebenfalls als Messinformationen zugeführt werden. Besonders bevorzugt wird dem Messgut mehr Temperiermedium zugeführt, wenn die Temperatur im Messgut niedriger als der Sollwert ist, und weniger Temperiermedium, wenn die Temperatur im Messgut höher als der Sollwert ist. Für den Betrieb der Regeleinrichtung und der Stellorgane ist in der Regel Hilfsenergie erforderlich, meist in Form von elektrischer Energie.

Es ist jedoch auch möglich, dass die Regelungseinrichtung sowie Stellorgan oder Stellorgane für das Temperiermedium ohne Hilfsenergie auskommen. Die Temperatur im Messgut wird in diesem Fall durch einen Thermostaten erfasst, der die Funktionen eines Temperatur-Messfühlers und einer Regeleinrichtung in einem Bauteil vereint. Der gewünschte Sollwert kann an dem Thermostaten eingestellt werden. Zwischen Thermostaten und Stellorgan oder Stellorganen besteht eine Wirkverbindung, beispielsweise mechanischer, hydraulischer oder pneumatischer Art, über die einer Temperaturabweichung zwischen Messguttemperatur und Sollwert entgegen gewirkt wird. Bei einer besonders bevorzugten Ausführungsform besteht die Wirkverbindung aus einem Rohr, das mit Flüssigkeit gefüllt ist. Als Stellorgane kommen bevorzugt Stellventile zum Einsatz. Steigt die Temperatur im Messgut, dehnt sich die Flüssigkeit vom Thermostaten in Richtung Stellventil aus und drückt im Stellventil, beispielsweise über einen Stellbalg, den Ventilkegel in Richtung des Ventilsitzes, was dazu führt, dass weniger Temperiermedium durch das Ventil strömen kann. Geht die Temperatur zurück, verringert sich das Volumen der Flüssigkeit und der Ventilkegel entfernt sich wieder von dem Ventilsitz, der Durchfluss an Temperiermedium steigt. Die Einstellung am Thermostaten wird so gewählt, dass bei Erreichen des gewünschten Temperatursollwertes, also einer verschwindenden Regelabweichung zwischen Temperatur des Messgutes und Sollwert, eine vorab berechnete Menge an Temperiermedium durch das Stellventil fließt.

Der oder die Temperatur-Messfühler oder der oder die Thermostate können an unterschiedlichen Stellen der erfindungsgemäßen Vorrichtung angebracht sein. In einer bevorzugten Ausführungsform ist mindestens ein Temperatur-Messfühler oder Thermostat in Fließrichtung des Messgutes hinter dem Einlass eines Temperiermediums angebracht, dessen Stellorgan durch den Messfühler oder Thermostat beeinflusst wird. Besonders bevorzugt befindet sich der Messfühler in der ersten Hälfte der Strecke zwischen dem Einlassstutzen eines Temperiermediums, dessen Stellorgan durch den Messfühler oder Thermostat beeinflusst wird, und dem Auslass des Messgutes. Der optimale Abstand und somit die Länge der Regelstrecke kann an die konkreten Strömungs- und Temperaturverhältnisse von Messgut und Temperiermedium angepasst werden.

In einer weiteren erfindungsgemäßen Ausführungsform wird ein Temperiermedium oder mehrere Temperiermedien dem Zulauf des Messgutes zugeführt. TemperaturMessfühler oder Thermostat können erfindungsgemäß im Zulauf des Messgutes oder am Grundelement des Strippers angebracht sein.

Für die Fertigung der Strippvorrichtung steht eine Vielzahl an Materialien prinzipiell zur Verfügung. Die Strippvorrichtung kann aus einem Material oder aus unterschiedlichen Materialien gefertigt sein. Beispiele für geeignete Materialien sind Stahl, Kunststoff, Glas oder auch Kombinationen daraus. Bevorzugt kommen Bauteile aus nichtrostenden Edelstählen, Tempergrauguss oder auch Kunststoffen wie Polyethylenterephthalat (PTFE) zum Einsatz. Abhängig von dem zu behandelnden Messgut können die erfindungsgemäße Vorrichtung oder Teile davon auch aus speziellen Legierungen hergestellt oder mit speziellen Beschichtungen versehen sein. Einige oder auch alle Bauteile der erfindungsgemäßen Strippvorrichtung können isoliert sein, um Wärmeverluste über die Wände der Bauteile zu verringern. Beispielsweise können Rohrleitungen ummantelt sein. Geeignete Isoliermaterialien sind dem Fachmann bekannt.

Das erfindungsgemäße Verfahren und die erfindungsgemäße Vorrichtung können, wie oben erwähnt, auf den unterschiedlichsten Gebieten zum Einsatz kommen. Sie finden vorteilhaft Anwendung auf Messgüter, aus denen sich eine oder mehrere Komponenten ausstrippen lassen. Bevorzugt handelt es sich bei dem Messgut im Wesentlichen um Wasser, das mit organischen oder anorganischen Verbindungen verunreinigt sein kann. Ein Anwendungsbereich des erfindungsgemäßen Verfahrens und der erfindungsgemäßen Vorrichtung betrifft die Kontrolle von behandlungsbedürftigem und nicht-behandlungsbedürftigem Abwasser oder Kondensat aus verfahrenstechnischen Anlagen. Ein weiterer Anwendungsbereich ist die Kontrolle von Wasser als Einsatzstoff zu verfahrenstechnischen Anlagen, insbesondere wenn hohe Reinheitsanforderungen an das Wasser als Einsatzstoff gestellt werden. Das Anwendungsfeld der Verfahrenstechnik ist nur als Beispiel anzusehen. Selbstverständlich können das erfindungsgemäße Verfahren und die erfindungsgemäße Vorrichtung auch auf anderen Gebieten Anwendungen finden, beispielsweise in der Kraftwerkstechnik, bei Kläranlagen, in biotechnologischen Anlagen oder in der Trinkwassergewinnung.

Die Liste der Beispiele ist nicht abschließend zu lesen. Prinzipiell lassen sich das erfindungsgemäße Verfahren und die erfindungsgemäße Vorrichtung bei allen Strippprozessen anwenden, bei denen eine effiziente und schnelle Temperierung eines Messgutes gewünscht und die Zufuhr von Temperiermedium in das Messgut möglich ist.

Anhand der Zeichnungen wird im Folgenden die Erfindung weiter erläutert, wobei die Zeichnungen als Prinzipdarstellungen zu verstehen sind. Sie stellen keine Beschränkung der Erfindung, beispielsweise im Hinblick auf konkrete Abmessungen oder Ausgestaltungsvarianten von Bauteilen des Strippers, dar. Es zeigen:
Fig. 1: Prinzipskizze eines von unten beschickten Gleichstrom-Rohrstrippers
Fig. 2: Prinzipskizze eines von oben beschickten Gleichstrom-Rohrstrippers
Fig. 3: Prinzipskizze eines Gegenstrom-Rohrstrippers
Fig. 4: Prinzipskizze eines Gegenstrom-Rohrstrippers mit zusätzlichem Gaszulauf
Fig. 5: Abhängigkeit eines FID-Signals von der Temperatur des Messgutes
Fig. 6: Abhängigkeit eines FID-Signals von den Durchflussraten an Messgut und Strippmedium für eine konstante Temperatur

### Liste der verwendeten Bezugszeichen

- 1: Stripper / Rohrstripper
- 10: Zulauf Messgut
- 11: Mischeinrichtung
- 20: Zulauf Temperiermedium
- 21: Stellorgan Temperiermedium
- 22: Einlassstutzen Temperiermedium
- 30: Zulauf Strippmedium
- 31: Stellorgan Strippmedium
- 32: Einlassstutzen Strippmedium
- 40: Auslass Messgut
- 41: Siphon
- 50: Auslass Messgas
- 60: Regeleinrichtung
- 61: Temperaturmessfühler
- 62: Temperaturmessfühler
- 63: Durchflussmessfühler
- 70: zusätzlicher Zulauf
- 71: Stellorgan zusätzlicher Zulauf

Gleichartige Einrichtungen, die mehrfach vorhanden sein können, werden zusätzlich mit den Buchstaben a, b, c, etc. bezeichnet, z.B. mehrere Zuläufe für Temperiermedien (20a, 20b).

In Fig. 1 ist ein Ausführungsbeispiel eines Rohrstrippers 1 dargestellt, bei dem das Messgut und das Strippmedium im Gleichstrom von unten nach oben strömen. Das Messgut wird über einen Zulauf 10 von unten in den Rohrstripper 1 eingebracht. Oberhalb des Messgut-Zulaufes 10 befindet sich der Zulauf 20 für ein Temperiermedium. Der Einlassstutzen 22 ist derart ausgestaltet, dass sich Messgut und Temperiermedium schnell vermischen, sodass das Messgut schnell und gleichmäßig temperiert wird. Zwischen Messgutzulauf 10 und Zulauf des Temperiermediums 20 oder bevorzugt oberhalb des Zulaufs des Temperiermediums 20 wird ein Strippmedium durch einen weiteren Zulauf 30 zugeführt. Durch die Zufuhr des gasförmigen Strippmediums werden Komponenten aus der Mischung aus Messgut und Temperiermedium ausgestrippt. Am oberen Ende des Strippers zweigen zwei Leitungen ab, wovon eine für die Ableitung des Messgutes und die andere für die Abführung des Messgases vorgesehen sind. Die Leitung zur Messgutabfuhr 40 ist in Form eines Siphons 41 ausgebildet. Durch die in dem Siphon sich befindende Flüssigkeit wird ein hydrostatischer Druck aufgebaut, der das Messgas daran hindert, den Rohrstripper durch den Siphon zu verlassen. Stattdessen strömt das Messgas durch den Messgas-Auslass 50 aus der Apparatur. Durch eine geeignete Dimensionierung des Siphons kann der Überdruck des Messgases so beeinflusst werden, dass das Messgas Strömungswiderstände im Leitungsweg zu den Analyseeinrichtungen überwinden kann. In einem solchen Fall kann auf eine Zwangsförderung des Messgases, beispielsweise durch Abpumpen oder Absaugen, verzichtet werden.

Vorteilhaft werden die Leitungen beheizt, durch die das Messgas vom Stripper bis zu den Analyseeinrichtungen strömt (in Fig. 1 nicht abgebildet), damit aus dem Messgas keine Komponenten durch Abkühlung auskondensieren, wodurch das Messergebnis verfälscht würde. Ein ähnlicher Effekt kann durch eine Isolierung der Leitungen, durch die das Messgas strömt, erzielt werden. Bei sehr kurzen Leitungen zwischen Messgas-Auslass 50 und Analyseeinrichtungen kann auf derartige Maßnahmen verzichtet werden.

Die Ausführung der Leitung zur Messgutabfuhr 40 in Form eines Siphons 41 ist nicht zwingend. Erfindungsgemäß muss lediglich sichergestellt werden, dass das Messgas nicht durch diese Leitung entweichen kann. Dies kann alternativ beispielsweise auch dadurch erreicht werden, dass durch Stellorgane im Zu- und/oder Ablauf des Messgutes der Füllstand des Messgutes im Stripper derart beeinflusst wird, dass der Flüssigkeitspegel oberhalb der Leitung zur Messgutabfuhr gehalten wird. Hierzu kann ein Messfühler, der den Füllstand erfasst, sowie eine Regeleinrichtung, die die jeweiligen Stellorgane im Zu- und/oder Ablauf des Messgutes beeinflusst, vorgesehen sein. Diese alternative Ausführungsform ist in den Zeichnungen nicht dargestellt.

Oberhalb des Zulaufs 20 für das Temperiermedium ist ein Messfühler 61 zur Erfassung der Temperatur des Messgutes angebracht. Es können noch weitere Temperaturmessfühler 62 vorgesehen werden. Die Messsignale des einen oder der mehreren Temperaturmessfühler 61, 62 werden einer Regeleinrichtung 60 zugeführt, die diese Daten mit einem vorgegebenen Sollwert für die Temperatur vergleicht und eine Regelabweichung berechnet. Auf Basis eines gegebenen Regelalgorithmus wird aus der Regelabweichung ein weiteres Signal von der Regeleinrichtung 60 erzeugt, das an ein Stellorgan 21 im Zulauf 20 des Temperiermediums geleitet wird. Ist die Temperatur im Messgut niedriger als der Sollwert, erhöht das Stellorgan 21 den Durchfluss an heißem Temperiermedium. Ist die Temperatur im Messgut höher als der Sollwert, wird der Durchfluss durch das Stellorgan 21 entsprechend reduziert.

Auch die Zufuhr an Strippmedium kann durch ein Stellorgan 31 beeinflusst werden. Der Einlass 32 des Strippmediums ist vorteilhaft derart gestaltet, dass das Strippmedium im Querschnitt möglichst gleichförmig durch das Messgut strömt. Dies kann auf unterschiedliche Arten geschehen, beispielsweise durch ein Rohr, das in die Flüssigphase des Strippers ragt und mit mehreren Löchern versehen ist, aus denen das Strippmedium strömt. Bevorzugt wird das Strippmedium über eine Fritte in den Stripper geleitet.

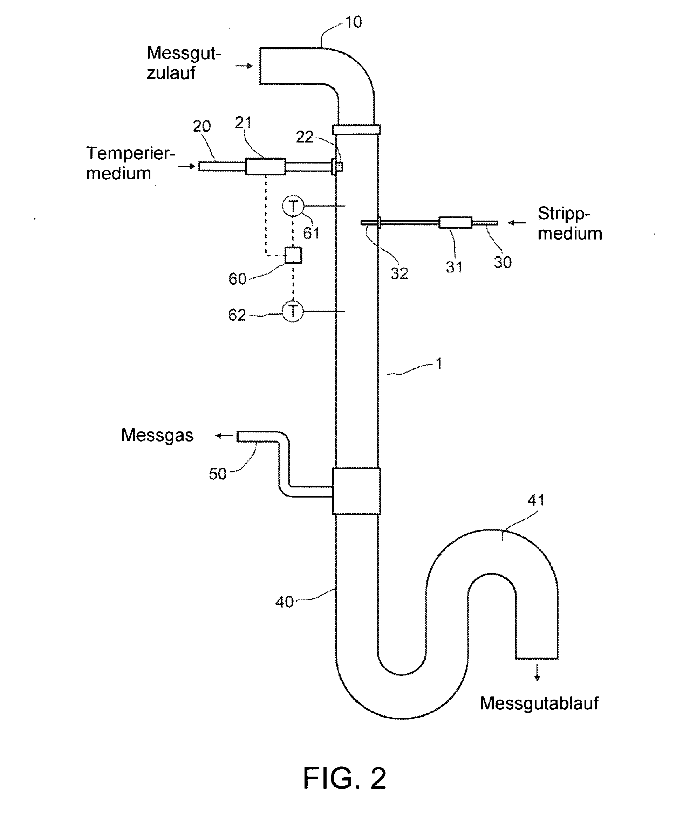

Fig. 2 zeigt ein Ausführungsbeispiel eines Strippers 1, bei dem Messgut und Strippmedium im Gleichstrom von oben nach unten strömen. Das Messgut wird über einen Zulauf 10 am oberen Ende dem Stripper zugeführt. Unterhalb des Messgut-Zulaufes 10 wird ein Temperiermedium über einen Zulauf 20 in den Stripper eingebracht. Wiederum unterhalb des Temperiermedium-Zulaufes 20 wird ein Strippmedium über einen Zulauf 30 zugeführt. Das untere Ende des Strippers 1 ist in Form eines Siphons 41 ausgestaltet, in dem sich flüssiges Messgut sammelt und für einen Füllstand an Messgut im Stripper 1 sorgt. Das Messgut verlässt den Stripper 1 durch den Siphon 41. Oberhalb der Höhe der oberen Siphonkrümmung befindet sich im Stripper 1 ein Auslass 50 für das Messgas. Der Flüssigkeitsstand im Stripper 1 sorgt dafür, dass das Messgas nicht durch den Siphon 41 entweicht. Vorteilhaft ist bei dieser Ausführungsform der Stripper teilweise mit Füllkörpern gefüllt, die die Oberfläche im Stripper 1 vergrößern, wodurch eine Intensivierung des Stoffaustausches zwischen Messgut und Strippmedium bewirkt wird. Das Strippmedium nimmt Komponenten aus der Mischung aus Messgut und Temperiermedium auf und verlässt als Messgas den Stripper 1. Bevorzugt unterhalb des Zulaufs 20 für das Temperiermedium ist in dem Stripper 1 ein Temperaturmessfühler 61 angebracht, der die Temperatur des Messgutes erfasst. Im Strömungsverlauf weiter unterhalb kann ein weiterer Temperaturmessfühler 62 angebracht sein. Die Signale des oder der Temperaturmessfühler werden einer Regelungseinrichtung 60 zugeleitet, in der diese Daten mit einem vorgegebenen Sollwert für die Temperatur verglichen werden. Auf Basis eines gegebenen Regelalgorithmus berechnet sich aus der Abweichung von Sollwert und Istwert der Temperatur ein Signal, das an ein Stellorgan 21 im Zulauf 20 des Temperiermediums geleitet wird. Ist die Temperatur im Messgut niedriger als der Sollwert, erhöht das Stellorgan 21 den Durchfluss an heißem Temperiermedium. Ist die Temperatur im Messgut höher als der Sollwert, wird der Durchfluss durch das Stellorgan 21 entsprechend reduziert.

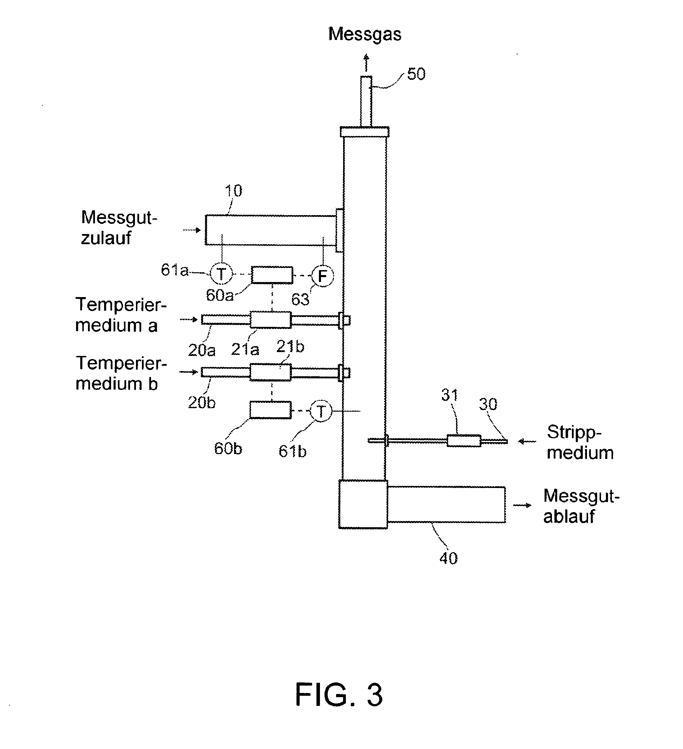

Fig. 3 stellt beispielhaft eine Ausführungsform eines Rohrstrippers 1 dar, bei dem Messgut und Strippmedium im Gegenstrom geführt werden. Das Messgut wird über einen Zulauf 10 in den oberen Teil des Strippers geleitet und strömt nach unten. Unterhalb des Messgut-Zulaufes 10 ist ein erster Zulauf 20a für ein Temperiermedium angebracht. Ein erster Messfühler 61a erfasst die Temperatur des zugeführten Messgutes im Zulauf 10, ein weiterer Messfühler 63 den Durchfluss des Zulaufs. In einer ersten Regeleinrichtung 60a werden die Signale des ersten Temperaturmessfühlers 61a und des Durchflussmessfühlers 63 in einem Regelalgorithmus so verarbeitet, dass der Bedarf an Temperiermedium bestimmt wird, der zu Aufheizung des Messgut-Zulaufs erforderlich ist. Ein entsprechendes Signal wird von der ersten Regeleinrichtung 60a an das Stellorgan 21 a des ersten Zulaufs des Temperiermediums geleitet. Unterhalb des ersten Zulaufs 20a kann ein zweiter Zulauf 20b für das Temperiermedium vorgesehen sein. Ein weiterer Messfühler 61 b liefert ein Signal für die Temperatur des Messgutes im Stripper. In einer zweiten Regeleinrichtung 60b wird dieses Signal mit einem Sollwert für die Temperatur verglichen. Ist die Temperatur zu niedrig, gibt die Regeleinrichtung 60b ein Signal an ein Stellorgan 21 b im zweiten Zulauf 20b, das daraufhin den Durchfluss an Temperiermedium in den Stripper erhöht. Ist die Temperatur an dem Messfühler 61 b zu hoch, wird der Durchfluss durch das Stellorgan 21 b verringert.

Die unterschiedlichen .Regeleinrichtungen 60a, 60b können auch in einer gemeinsamen Regeleinrichtung realisiert werden, die sämtliche Messsignale empfängt und auf Basis eines Algorithmus Signale für die Stellorgane 21 a, 21 b ermittelt und weiterleitet. Regeleinrichtungen mit derartigen Algorithmen werden auch als MIMO-Regler (multiple input, multiple output) bezeichnet. Am unteren Ende des Rohrstrippers 1 wird das Strippmedium über einen Zulauf 30 zugeführt und strömt von unten nach oben durch den Stripper. Dabei werden Komponenten aus dem von oben nach unten fließenden Gemisch aus Messgut und Temperiermedium ausgestrippt und verlassen zusammen mit dem Strippmedium als Messgas durch einen Auslass 50 am oberen Ende den Stripper. Das Messgut verlässt den Stripper durch einen Auslass 40 am unteren Ende.

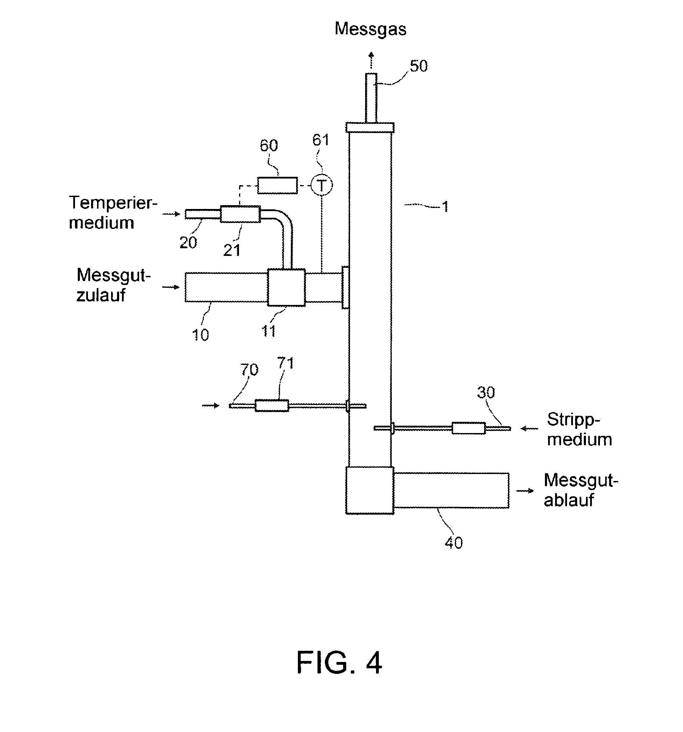

Fig. 4 zeigt eine weitere Ausführungsform eines Gegenstrom-Rohrstrippers. Analog zu Fig. 3 wird das Messgut im oberen Teil des Strippers zugeführt. Die Temperierung des Messgutes erfolgt nun allerdings vor dem Eintritt in den Stripper. Dazu ist eine Mischeinrichtung 11 im Zulauf 10 des Messgutes vorgesehen, in die neben dem Messgut auch das Temperiermedium eingebracht wird. Ein Messfühler 61 ermittelt die Mischtemperatur nach der Mischeinrichtung und leitet ein Messsignal an eine Regeleinrichtung 60. Durch Vergleich mit einem vorgegebenen Sollwert berechnet ein Regelalgorithmus daraus ein Signal für ein Stellorgan 21 im Zulauf 20 des Temperiermediums. Ist die Mischtemperatur zu niedrig, wird der Durchfluss des Temperiermediums erhöht, ist sie zu hoch, wird der Durchfluss verringert.

Analog zu der Ausführungsform in Fig. 3 wird am unteren Ende des Strippers ein Strippmedium durch einen Zulauf 30 in das Messgut eingebracht und strömt nach oben, entgegen der Fließrichtung des Messgutes, das den Stripper am unteren Ende durch einen Auslass 40 verlässt. Zusätzlich zu dem Strippmedium können weitere Substanzen durch einen oder mehrere weitere Zuläufe 70 in den Stripper eingebracht werden. Diese Substanzen können beispielsweise als Referenzsubstanzen für ein Analyseverfahren oder zur Kalibrierung in einer Analyseeinrichtung Verwendung finden, z.B. als interner Standard in der Chromatographie. Der Zulauf oder die Zuläufe 70 können an unterschiedlichen Stellen am Stripper 1 oder auch am Zulauf des Messgutes 10, beispielsweise an der Mischeinrichtung 11, angebracht sein. Es muss lediglich sichergestellt sein, dass die Substanzen mit dem Messgas den Stripper verlassen und zu den Analyseeinrichtungen geleitet werden. Im Fall der Einbringung der zusätzlichen Substanzen in das Messgut beispielsweise ist dies dann gegeben, wenn die Substanzen durch das Strippmedium teilweise oder vollständig in das Messgas ausgestrippt werden. Die Substanzen können aber z.B. auch gasförmig in die Gasphase des Strippers oder direkt in das Messgas eingeleitet werden. Der Zulauf oder die Zuläufe 70 können mit einem Stellorgan 71 versehen sein, um den Durchfluss zu beeinflussen.

Auch wenn nur in der Fig. 4 ein zusätzlicher Zulauf 70 dargestellt ist, so können selbstverständlich auch bei anderen Ausführungsformen Zuläufe 70 für weitere Substanzen angebracht werden.

Der Abschnitt der Strippvorrichtung, in der Stoffaustausch zwischen Gas- und Flüssigphase stattfindet, kann mit Einbauten versehen sein, um den Stoffaustausch zu fördern. Diese können fest installierte Einbauten sein. Es ist aber auch möglich, dass es sich dabei um lose Einbauten handelt. Beispiele für entsprechende Einbauten sind Leitbleche oder Turbulatoren, die für eine Verwirbelung insbesondere der Flüssigkeitsströmung sorgen. Weiterhin können geordnete oder ungeordnete Packungen oder Füllkörperschüttungen eingebracht sein, wie sie z.B. aus der Destillations- oder Absorptionstechnik bekannt sind.

Die Zufuhr von Temperiermedium kann die einzige Wärmequelle sein, es können aber auch zusätzliche Wärmequellen vorhanden sein. Beispielsweise kann der Stripper mit einem Heizmantel versehen sein, der als Doppelrohr ausgeführt ist. Messgut, Temperiermedium und Strippmedium strömen dabei im Innenrohr, währen ein Heizmedium durch das Außenrohr fließt. Geeignete Heizmedien können heißes Wasser, heißes Produkt oder auch Dampf sein. Anstelle eines Doppelrohres kann der Stripper auch auf seiner Außenseite mit einer Rohrschlange oder einer Wendel aus Halbrohren versehen sein, durch die ein Heizmedium fließt und Wärme über die Wand an die Gas- und Flüssigphase im Stripper abgibt. Wird eine solche zusätzliche Wärmequelle verwendet, so leistet sie den Grundbeitrag zur Erwärmung des Messgutes. Die Temperierung auf den gewünschten Sollwert der Temperatur im Messgut wird wie bei der Variante ohne zusätzliche Wärmequelle durch die Zufuhr eines Temperiermediums in das Messgut realisiert.

Ein Vorteil der Erfindung ist darin zu sehen, dass mittels des erfindungsgemäßen Verfahrens bzw. bei Einsatz der erfindungsgemäßen Vorrichtung Temperaturschwankungen, beispielsweise im Zulauf des Messgutes, sehr schnell ausgeglichen werden können. Dadurch kann eine im Wesentlichen gleichmäßige Temperatur im Messgut und somit im Messgas zu den Analyseeinrichtungen sichergestellt werden. Weiterhin wird durch eine Erhöhung der Temperatur im Messgut das Spektrum der nachweisbaren Substanzen erweitert. Insbesondere wenn Prozessdampf in verfahrenstechnischen Anlagen zur Verfügung steht, weist das erfindungsgemäße Verfahren darüber hinaus deutliche Kostenvorteile gegenüber Alternativen wie elektrischer Beheizung auf.

### Beispiel

### Versuchsaufbau

Der Aufbau der verwendeten Strippeinrichtung ist schematisch in Fig. 1 dargestellt. Das Messgut gelangt mittels einer Probenpumpe oder unter Nutzung des hydrostatischen Drucks von unten in den Stripper 1, Der Messgutfluss wird dabei durch einen vorgeschalteten Magnetisch-Induktiven Durchflussmesser (MID) überwacht. Etwa 30 cm oberhalb des Messgutzulaufes 10 befindet sich ein horizontal eingebauter Dampfinjektor 22, der Wasserdampf mit einer Temperatur von ca. 130°C direkt in das Messgut injiziert und dieses auf die gewünschte Temperatur erhitzt. Der für die Temperaturregelung eingesetzte Fühler 61 befindet sich im Abstand von ca. 20 cm oberhalb des Injektors. Gereinigte Luft wird als Strippmedium ca. 40 cm oberhalb des Dampfinjektors in den Stripper zügeführt 30 und im Gleichstrom mit dem Messgut nach oben geführt. Die Strecke vom Ort des Einlasses 32 der Strippluft bis zum oberen Ende des Strippers ist ca. 50 cm lang und bildet den Phasentauscher, in dem sich die Strippluft mit den aus dem Messgut ausgetriebenen Komponenten anreichert. Ein zweiter Temperaturfühler 62 befindet sich ca. 20 cm oberhalb des Stripplufteinlasses 32 und dient als Temperaturüberwachung für die Sicherheitsschaltung.

Das Messgut verlässt den Stripper am oberen Ende über einen Auslass 40 und gelangt durch einen Siphon 40 ins Abwasser. Die mit aus dem Messgut ausgestrippten Komponenten angereicherte Strippluft verlässt im höchsten Punkt den Stripper als Messgas. Durch eine Wassersäule von ca. 30 cm Höhe im Siphon wird ein Überdruck von ca. 30 mbar generiert, der dafür sorgt, dass das Messgas nicht durch den Siphon abströmen kann. Der Überdruck, der durch ein Manometer überwacht werden kann, ist so ausgelegt, dass Strömungswiderstände der nachgeschalteten Probenaufbereitung vom Messgas überwunden werden. Solche Widerstände ergeben sich in der konkreten Versuchsanordnuhg durch ein Filter zur Rückhaltung von Aerosolen und ein Durchflussmessgeräte im Strömungsweg des Messgases. Die Messgasleitung vom Stripper bis zu den Analyseeinrichtungen ist durchgängig beheizt und isoliert. Die Temperatur ist so gewählt, dass das Messgas nicht kondensiert, sie also mindestens der Temperatur am Austritt aus dem Stripper entspricht.

Als Analyseeinrichtung wird ein Flammen-Ionisationsdektor (FID) eingesetzt, in dem das Messgas auf die Summe alle kohlenwasserstoffhaltigen Verbindungen analysiert wird. Das Messsignal wird über verschiedene Ausgänge (analog und digital) zur weiteren Verarbeitung bereitgestellt.

### Versuchsbedingungen

Als Versuchssubstanz, die in Wasser nachgewiesen werden soll, wurde Tetrahydrofuran (THF) ausgewählt. THF ist in Wasser gut löslich und lässt sich schwer, aber reproduzierbar ausstrippen.

Das Messgut wird mit einer Pumpe aus einem 100-Liter-Fass zum Strippsystem gefördert. Das Messgut wird durch direkte Injizierung von Wasserdampf auf eine bestimmte Endtemperatur temperiert, die enthaltenen Kohlenwasserstoffe mit Luft ausgestrippt und mit einem FID detektiert. Ein Siemens-Schreiber erfasst das FID-Messsignal und die Temperatur des Messgutes. Die Dampfmenge wird am Dampfmengenmesser, die Messgutmenge am MID und die Strippluftmenge am Strömungsmesser direkt abgelesen und erfasst. Folgende Geräte wurden verwendet:
- Testa FID 123 kalibriert auf Propan (Prüfgas mit 800 ppm Propan vor jeder Messreihe); FID-Messbereiche (MB): MB1 = 10 ppm; MB2 = 200 ppm; MB3 = 1000 ppm (bezogen jeweils auf Propan); Probemenge zum FID: 14 ml/min
- MID kalibriert auf 0-200 l/h Wasser
- ABB Kopfmessumformer mit Pt100-Temperaturfühler 0-100°C kalibriert
- Messgasweg durchgehend auf mindestens 120°C beheizt

### Versuch 1

Zunächst wurde untersucht, welche Dampfmengen nötig sind, um das Messgut auf eine bestimmte Temperatur zu erwärmen. Als Messgut wurde Wasser mit einem THF-Gehalt von 40 ppm bei 15°C verwendet. Der Messgutstrom betrug 60 l/h und wurde während des Versuchs konstant gehalten. In der Tabelle 1 sind die erforderlichen Mengen an Dampf (4 bar absolut, ca. 130 °C) für die jeweilige Mischtemperatur angegeben.

**Tabelle 1**

| | | | | | | |
|---|---|---|---|---|---|---|
| Mischtemperatur [°C] | 15 | 30 | 40 | 50 | 60 | 67 |
| Dampfstrom [kg/h] | 0 | 1,3 | 2,1 | 3,2 | 4,3 | 5,7 |

### Versuch 2

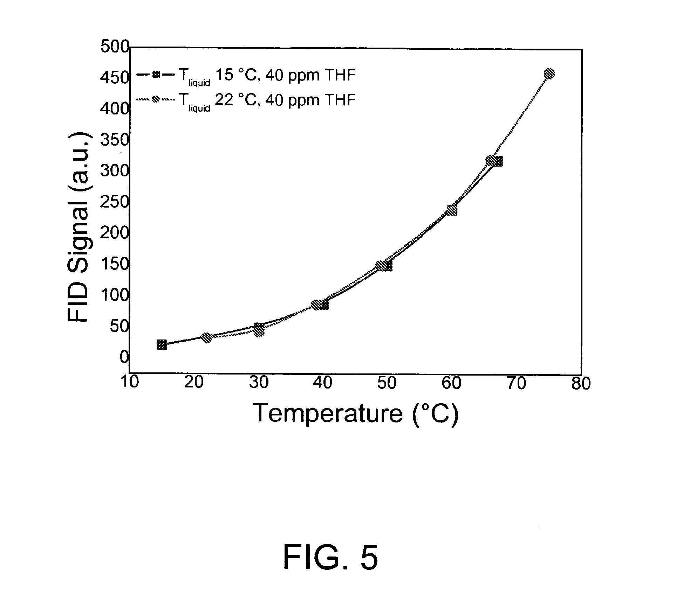

In einem weiteren Versuch wurde das Messgut mit derselben Zusammensetzung wie in Beispiel 1 mit einem Strom von 60 l/h in den Stripper gepumpt. Als Strippmedium wurde gereinigte Luft mit einer Flussrate von 80 l/h eingesetzt. Untersucht wurde die Abhängigkeit des FID-Signals von der Temperatur des Messgutes. Dazu wurde analog zu Beispiel 1 Wasserdampf (4 bar absolut, ca. 130 °C) in das Messgut eingebracht, um die jeweilige Temperatur einzustellen. Fig. 5 zeigt die Ergebnisse für zwei unterschiedliche Fälle: Im ersten Fall (Quadrat) wurde das Messgut mit 15°C in den Stripper gepumpt, im zweiten Fall (Kreis) mit 22°C. Wie oben beschrieben ist der Maximalwert an Temperiermedium, das dem Stripper zugeführt werden kann, unter anderem durch die Druckdifferenz zwischen Stripper und Quelle des Temperiermediums begrenzt. Im vorliegenden Fall stammte der als Temperiermedium eingesetzte Dampf aus einem Dampfnetz mit annähernd konstantem Druck von 4 bar absolut. Bei einem Absolutdruck von ca. 1 bar im Stripper betrug der maximal zur Verfügung stehende Einsatzstrom an Dampf 6 kg/h. Aufgrund dieser Begrenzung ließ sich das Messgut im ersten Fall nicht so weit erwärmen wie im zweiten Fall. Wie aus Fig. 5 deutliche hervorgeht, ist die Höhe des FID-Signals (willkürliche Einheit, "arbitrary unit") stark von der Temperatur des Messgutes abhängig. Weiterhin ist erkennbar, dass die Eintrittstemperatur des Messgutes für das Messgut kaum eine Rolle spielt. Obwohl zur Erwärmung des kälteren Messgutes mehr Dampf erforderlich ist, um dieselbe Temperatur einzustellen, sind die jeweiligen Signale, die bei dieser Temperatur erhalten werden, nahezu identisch. Der Verdünnungseffekt durch die unterschiedlich großen zugeführten Dampfmengen ist demnach vernachlässigbar.

### Versuch 3

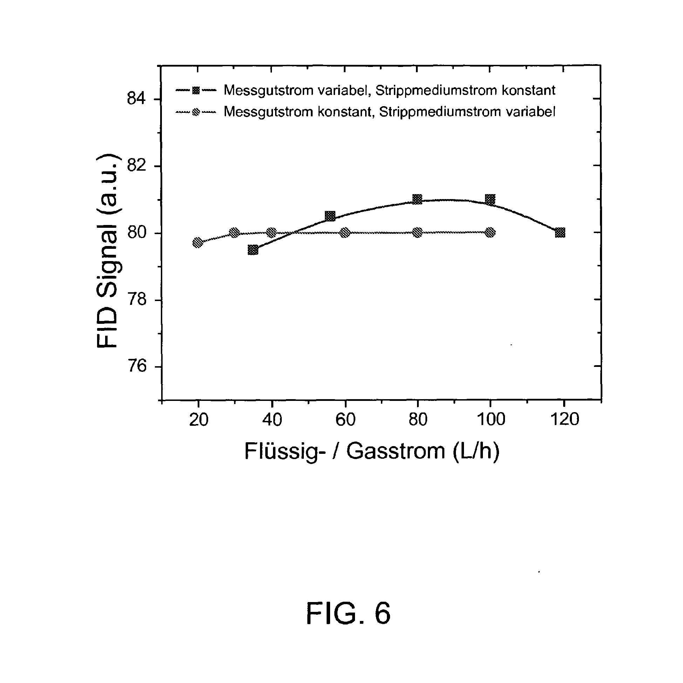

Untersucht wurde der Einfluss einer Variation der Flussraten an Messgut und an Strippmedium. Dazu wurde wiederum ein Messgut mit derselben Zusammensetzung wie in den vorherigen Beispielen in den Stripper gepumpt und durch Einbringen von Wasserdampf auf eine konstante Temperatur von 40°C temperiert. Fig. 6 zeigt die Abhängigkeit des FID-Signals (willkürliche Einheit, "arbitrary unit") für zwei unterschiedliche Szenarien: Zunächst wurde der Messgutfluss auf 60 l/h konstant gehalten und die Zufuhr an Strippluft von 20 l/h bis 100 l/h variiert. Die erhaltenen FID-Signale sind in Fig. 6 mit einem Kreis gekennzeichnet. Anschließend wurde der Zufluss an Strippluft auf 80 l/h konstant eingestellt und die Menge an zugeführtem Messgut von 35 l/h bis 120 l/h variiert. Die Kurve der entsprechenden FID-Signale sind in Fig. 6 mit einem Quadrat markiert. Es ist deutlich zu erkennen, dass trotz signifikanter Änderung der Durchflüsse an Messgut und Strippmedium sich das resultierende FID-Signal in einem sehr engen Bereich (79 bis 81) bewegt.

## Patentansprüche

1. Verfahren zur Analyse eines Messgutes, wobei das Messgut einem Stripper (1) zugeführt wird, mindestens ein gasförmiges Strippmedium in das Messgut eingebracht wird, Komponenten zumindest teilweise aus dem Messgut in das Strippmedium übergehen und das so erhaltene Messgas in eine Analyseeinrichtung geleitet wird, **dadurch gekennzeichnet, dass** das Messgut temperiert wird, indem Dampf mindestens eines Temperiermediums direkt in das Messgut eingebracht wird.

2. Verfahren nach Anspruch 1, **dadurch gekennzeichnet, dass** die Temperatur des Messgutes durch mindestens einen Messfühler (61) erfasst wird und eine Regeleinrichtung (60) auf der Basis eines Vergleichs der Messguttemperatur mit einem vorgegebenen Sollwert mindestens ein Stellorgan (21) in mindestens einem Zulauf (20) des mindestens einen Temperiermediums so beeinflusst, dass das Stellorgan (21) einer Abweichung zwischen Messguttemperatur und Sollwert entgegenwirkt.

3. Verfahren nach Anspruch 1, **dadurch gekennzeichnet, dass** die Temperatur im Messgut durch einen Thermostaten erfasst wird, dem ein Sollwert für die Temperatur vorgegeben ist und der weiterhin mit einem Stellorgan (21) in Wirkverbindung steht, sodass das Stellorgan (21) einer Temperaturabweichungzwischen Messguttemperatur und Sollwert entgegenwirkt.

4. Verfahren nach Anspruch 2 oder 43, **dadurch gekennzeichnet, dass** mindestens ein Messfühler (61) oder Thermostat in Fließrichtung des Messguts in der ersten Hälfte der Strecke zwischen einem Einlassstutzen (22) des mindestens einen Temperiermediums, dessen Stellorgan (21) durch den Messfühler (61) oder Thermostat beeinflusst wird, und einem Auslass (40) des Messgutes angebracht ist.

5. Verfahren nach einem der Ansprüche 1 bis 4, **dadurch gekennzeichnet, dass** in das Messgut oder Messgas mindestens eine weitere Substanz eingebracht wird, die in der Analyseeinrichtung als Referenzsubstanz, zur Markierung oder
Kalibrierung Verwendung findet.

6. Verfahren nach einem der Ansprüche 1 bis 5, **dadurch gekennzeichnet, dass** es sich bei dem Messgut um Abwasser handelt.

7. Verfahren nach Anspruch 6, **dadurch gekennzeichnet, dass** es sich bei dem Temperiermedium um Wasserdampf handelt.

8. Vorrichtung zum Strippen (1) von Komponenten aus einem 9.Vorrichtung nach Anspruche 8, wobei der mindestens eine weitere Einlass (20) als Düse oder Dampfinjektar ansgestaltet Messgut, umfassend ein Grundelement mit mindestens einem Einlass (10) und mindestens einem Auslass (40) für das Messgut, mindestens einem Einlass (30) für mindestens ein gasförmiges Strippmedium sowie, mindestens einem Auslass (50) für Messgas, das sich aus Strippmedium und aus dem Messgut ausgestrippten Komponenten zusammensetzt, und eine Analyseeinrichtung, in die das Messgas geleitet wird, **dadurch gekennzeichnet, dass** das Grundelement mindestens einen weiteren Einlass (20) für die direkte Einbringung eines Temperiermediums oder mehrerer Temperiermedien in das Messgut und mindestens einem Messfühler (61) zur Aufnahme der Temperatur des Messgutes umfasst.

9. Vorrichtung nach Anspruch 8, wobei der mindestens eine weitere Einlass (20) als Düse oder Dampfinjektor ausgestaltet ist.

10. Vorrichtung nach Anspruch 8 oder 9, wobei die Vorrichtung weiterhin eine Regeleinrichtung (60) umfasst, die auf Basis eines Vergleichs der Messguttemperatur mit einem vorgegebenen Sollwert mindestens ein Stellorgan (21) in einem Zulauf (20) des einen Temperiermediums oder der mehreren Temperiermedien beeinflusst.

11. Vorrichtung nach Anspruch 10, wobei der Messfühler (61) und die Regeleinrichtung (60) in Form eines Thermostaten realisiert sind, der mit dem Stellorgan (21) im Zulauf (20) des einen Temperiermediums oder der mehreren Temperiermedien in Wirkverbindung steht.

12. Vorrichtung nach einem der Ansprüche 8 bis 11, wobei das Grundelement im Wesentlichen aus einem rohrförmigen Bauteil besteht, an dessen unterem Ende der Einlass (10) für das Messgut vorgesehen ist, oberhalb des Messguteinlasses (10) der mindestens eine Einlass (20) für das eine Temperiermedium oder die mehreren Temperiermedien vorhanden ist, oberhalb des Temperiermedium-Einlasses (20) das Strippmedium durch den mindestens einen Einlass (30) eingebracht wird, am oberen Ende des rohrförmigen Bauteils eine Leitung zur Messgutabfuhr in Form eines Siphons (41) ausgebildet ist, und am obersten Ende des rohrförmigen Bauteils der Auslass (50) für Messgas vorhanden ist.

## Claims

1. A method for analyzing a measurement product, the measurement product being delivered to a stripper (1), at least one gaseous stripping medium being introduced into the measurement product, components being transferred at least partially out of the measurement product into the stripping medium, and the measurement gas thus obtained being conducted into an analyzer, wherein the measurement product is temperature-controlled in that steam from at least one temperature control medium is introduced directly into the measurement product.

2. The method according to claim 1, wherein the temperature of the measurement product is detected by at least one measurement sensor (61), and, on the basis of a comparison of the measurement product temperature with a stipulated desired value, a regulating device (60) influences at least one actuating member (21) in at least one inflow (20) of the at least one temperature control medium, such that the actuating member (21) counteracts a deviation between the measurement product temperature and the desired value.

3. The method according to claim 1, wherein the temperature in the measurement product is detected by a thermostat for which a desired value for the temperature is stipulated and which, furthermore, is operatively connected to an actuating member (21), so that the actuating member (21) counteracts a temperature deviation between the measurement product temperature and the desired value.

4. The method according to claim 2 or 3, wherein at least one measurement sensor (61) or thermostat is mounted in the direction of flow of the measurement product in the first half of the section between an inlet connection piece (22) for the at least one temperature control medium, the actuating member (21) of which is influenced by the measurement sensor (61) or thermostat, and an outlet (40) for the measurement product.

5. The method according to one of claims 1 to 4, wherein at least one further substance is introduced into the measurement product or measurement gas and is used in the analyzer as a reference substance for marking or calibration.

6. The method according to one of claims 1 to 5, wherein the measurement product is waste water.

7. The method according to claim 6, wherein the temperature control medium is steam.

8. An apparatus for stripping (1) components out of a measurement product, comprising a basic element with at least one inlet (10) and with at least one outlet (40) for the measurement product, with at least one inlet (30) for at least one gaseous stripping medium and with at least one outlet (50) for measurement gas which is composed of stripping medium and of components stripped out of the measurement product, and an analyzer into which the measurement gas is conducted, wherein the basic element comprises at least one further inlet (20) for the direct introduction of a temperature control medium or of a plurality of temperature control media into the measurement product and at least one measurement sensor (61) for recording the temperature of the measurement product.

9. The apparatus according to claim 8, the at least one further inlet (20) being configured as a nozzle or steam injector.

10. The apparatus according to claim 8 or 9, the apparatus comprising, furthermore, a regulating device (60) which, on the basis of a comparison of the measurement product temperature with a stipulated desired value, influences at least one actuating member (21) in an inflow (20) of the one temperature control medium or of the plurality of temperature control media.

11. The apparatus according to claim 10, the measurement sensor (61) and the regulating device (60) being implemented in the form of a thermostat which is operatively connected to the actuating member (21) in the inflow (20) of the one temperature control medium or of the plurality of temperature control media.

12. The apparatus according to one of claims 8 to 11, the basic element being composed essentially of a tubular component, at the lower end of which the inlet (10) for the measurement product is provided, the at least one inlet (20) for the one temperature control medium or the plurality of temperature control media being present above the measurement product inlet (10), the stripping medium being introduced through the at least one inlet (30) above the temperature control medium inlet (20), a line for measurement product discharge being designed in the form of a siphon (41) at the upper end of the tubular component, and the outlet (50) for measurement gas being present at the uppermost end of the tubular component.

## Revendications

1. Procédé d'analyse d'un milieu à mesurer, le milieu à mesurer étant introduit dans un dispositif d'extraction (1), au moins un agent d'extraction gazeux étant introduit dans le milieu à mesurer, des composants passant au moins en partie du milieu à mesurer dans l'agent d'extraction et le gaz à mesurer ainsi obtenu étant introduit dans un dispositif d'analyse, **caractérisé en ce que** le milieu à mesurer est conditionné en introduisant directement de la vapeur d'au moins un agent de conditionnement dans le milieu à mesurer.

2. Procédé selon la revendication 1, **caractérisé en ce que** la température du milieu à mesurer est déterminée par au moins un détecteur (61) et un dispositif de régulation (60) influence au moins un actionneur (21) dans au moins une alimentation (20) du ou des agents de conditionnement sur la base d'une comparaison de la température du milieu à mesurer avec une valeur prescrite fixée, de manière à ce que l'actionneur (21) contre une déviation entre la température du milieu à mesurer et la valeur prescrite.

3. Procédé selon la revendication 1, **caractérisé en ce que** la température dans le milieu à mesurer est déterminée par un thermostat, pour lequel une valeur prescrite pour la température est fixée et qui est également connecté opérationnellement avec un actionneur (21), de manière à ce que l'actionneur (21) contre une déviation de température entre la température du milieu à mesurer et la valeur prescrite.

4. Procédé selon la revendication 2 ou 3, **caractérisé en ce qu'**au moins un détecteur (61) ou un thermostat est placé dans la direction d'écoulement du milieu à mesurer dans la première moitié de la section entre une tubulure d'entrée (22) du ou des agents de conditionnement, dont l'actionneur (21) est influencé par le détecteur (61) ou le thermostat, et une sortie (40) du milieu à mesurer.

5. Procédé selon l'une quelconque des revendications 1 à 4, **caractérisé en ce qu'**au moins une substance supplémentaire est introduite dans le milieu à mesurer ou le gaz à mesurer, qui est utilisée en tant que substance de référence dans le dispositif d'analyse, pour le marquage ou pour l'étalonnage.

6. Procédé selon l'une quelconque des revendications 1 à 5, **caractérisé en ce que** le milieu à mesurer est une eau usée.

7. Procédé selon la revendication 6, **caractérisé en ce que** l'agent de conditionnement est de la vapeur d'eau.

8. Dispositif d'extraction (1) de composants d'un milieu à mesurer, comprenant un élément de base ayant au moins une entrée (10) et au moins une sortie (40) pour le milieu à mesurer, au moins une entrée (30) pour au moins un agent d'extraction gazeux, ainsi qu'au moins une sortie (50) pour le gaz à mesurer, qui est composé par l'agent d'extraction et les composants extraits du milieu à mesurer, et un dispositif d'analyse dans lequel le gaz à mesurer est conduit, **caractérisé en ce que** l'élément de base comprend au moins une entrée supplémentaire (20) pour l'introduction directe d'un agent de conditionnement ou de plusieurs agents de conditionnement dans le milieu à mesurer et au moins un détecteur (61) pour l'enregistrement de la température du milieu à mesurer.

9. Dispositif selon la revendication 8, dans lequel la ou les entrées supplémentaires (20) sont conçues sous la forme d'une buse ou d'un injecteur de vapeur.

10. Dispositif selon la revendication 8 ou 9, dans lequel le dispositif comprend également un dispositif de régulation (60) qui influence au moins un actionneur (21) dans une alimentation (20) de l'agent de conditionnement ou des agents de conditionnement sur la base d'une comparaison de la température du milieu à mesurer avec une valeur prescrite fixée.

11. Dispositif selon la revendication 10, dans lequel le détecteur (61) et le dispositif de régulation (60) sont réalisés sous la forme d'un thermostat, qui est connecté opérationnellement avec l'actionneur (21) dans l'alimentation (20) de l'agent de conditionnement ou des agents de conditionnement.

12. Dispositif selon l'une quelconque des revendications 8 à 11, dans lequel l'élément de base est essentiellement constitué par un élément tubulaire, à l'extrémité inférieure duquel l'entrée (10) pour le milieu à mesurer est prévue, la ou les entrées (20) pour l'agent de conditionnement ou les agents de conditionnement se trouvent au-dessus de l'entrée pour le milieu à mesurer (10), l'agent d'extraction est introduit par la ou les entrées (30) au-dessus de l'entrée pour l'agent de conditionnement (20), une conduite pour le déchargement du milieu à mesurer sous la forme d'un siphon (41) est formée à l'extrémité supérieure de l'élément tubulaire, et la sortie (50) pour le gaz à mesurer se trouve à l'extrémité la plus supérieure de l'élément tubulaire.
